# EUROPEAN PATENT SPECIFICATION

(11) **EP 2 809 392 B1**
(45) Date of publication and mention of the grant of the patent: **14.09.2016**
(21) Application number: 13703982.2
(22) Date of filing: 31.01.2013
(51) Int. Cl.: A61N 1/362, G01N 33/68

(54) **IMPLANTABLE DEVICE AND METHODS FOR DIAGNOSING HEART FAILURE USING BIOMARKER PANEL DATA**
IMPLANTIERBARES GERÄT UND VERFAHREN ZUR HERZFEHLER DIAGNOSE MITTLES BIOMARKER DATEN
DISPOSITIF IMPLANTABLE ET PROCÉDÉS DE DIAGNOSTIC D'UNE INSUFFISANCE CARDIAQUE SUR LA BASE DES DONNÉES ISSUES D'UN PANEL DE BIOMARQUEURS

(30) Priority: 31.01.2012 US 201261593046 P
(43) Date of publication of application: 10.12.2014
(73) Proprietor: CARDIAC PACEMAKERS, INC., St. Paul, MN 55122 (US); Musc Foundation for Research Development, Charleston, SC 29425 (US)
(72) Inventor: STOLEN, Craig M., New Brighton, MN 55112 (US); MEYER, Timothy E., North Oaks, MN 55127 (US); SETH, Milan, Minneapolis, MN 55408 (US); SPINALE, Francis G., Blythewood, SC 29016 (US); WOLD, Nicholas David, Arden Hills, MN 55112 (US)
(74) Representative: Pfenning, Meinig & Partner mbB
(86) International application number: PCT/US2013/024189
(87) International publication number: WO 2013/116547

(56) References cited:
- US-A1- 2006 258 952
- US-A1- 2011 295 084
- Aaron Glick ET AL: "Neurohormonal and inflammatory markers as predictors of short-term outcome in patients with heart failure and cardiac resynchronization therapy", IMAJ, 1 June 2006 (2006-06-01), pages 391-394, XP055060586, Retrieved from the Internet: URL:http://library.tasmc.org.il/Staff_Publ ications/publications 2006/glick.pdf [retrieved on 2013-04-22]
- FRANCISCO MARÍN ET AL: "Influence of cardiac resynchronization therapy on indices of inflammation, the prothrombotic state and tissue remodeling in systolic heart failure: A pilot study", THROMBOSIS RESEARCH, vol. 128, no. 4, 1 October 2011 (2011-10-01), pages 391-394, XP055060621, ISSN: 0049-3848, DOI: 10.1016/j.thromres.2011.05.022
- George N Theodorakis ET AL: "Antiinflammatory Effects of Cardiac Resynchronization Therapy in Patients with Chronic Heart Failure", PACE, 1 March 2006 (2006-03-01), pages 255-261, XP055060038, Retrieved from the Internet: URL:http://onlinelibrary.wiley.com/store/1 0.1111/j.1540-8159.2006.00331.x/asset/j.15 40-8159.2006.00331.x.pdf?v=1&t=hfmhqa3s&s= e64d599f69b957758a8219eee1202a966f281146 [retrieved on 2013-04-17]
- KUBANEK M ET AL: "Decrease in plasma B-type natriuretic peptide early after initiation of cardiac resynchronization therapy predicts clinical improvement at 12 months", EUROPEAN JOURNAL OF HEART FAILURE, ELSEVIER, AMSTERDAM, NL, vol. 8, no. 8, 1 December 2006 (2006-12-01), pages 832-840, XP028041807, ISSN: 1388-9842, DOI: 10.1016/J.EJHEART.2006.02.006 [retrieved on 2006-12-01]
- A ELMENYAR: "Cytokines and Myocardial Dysfunction: State of the Art", JOURNAL OF CARDIAC FAILURE, vol. 14, no. 1, 1 February 2008 (2008-02-01), pages 61-74, XP055060065, ISSN: 1071-9164, DOI: 10.1016/j.cardfail.2007.09.006
- K. A. ELLENBOGEN ET AL: "Primary Results From the SmartDelay Determined AV Optimization: A Comparison to Other AV Delay Methods Used in Cardiac Resynchronization Therapy (SMART-AV) Trial: A Randomized Trial Comparing Empirical, Echocardiography-Guided, and Algorithmic Atrioventricular Delay Programming in Cardiac Resynchron", CIRCULATION, vol. 122, no. 25, 12 November 2010 (2010-11-12), pages 2660-2668, XP055060712, ISSN: 0009-7322, DOI: 10.1161/CIRCULATIONAHA.110.992552

## Description

### TECHNICAL FIELD

This disclosure relates generally to medical devices and methods, and more particularly, to systems and methods for utilizing biomarker panel data with respect to medical devices and methods, amongst other things.

### BACKGROUND OF THE INVENTION

Implantable medical devices (IMDs) are commonly used to provide treatment to patients. Some types of implantable medical devices deliver electrical stimuli to a target tissue via a lead wire ("stimulation lead") or catheter having one or more electrodes disposed in or about the target tissue. In the context of cardiac rhythm management devices, the electrical stimuli can be delivered in the form of pacing pulses to pace the heart and/or relatively high energy defibrillation shocks or cardioversion shocks to terminate arrhythmias.

Cardiac resynchronization therapy (CRT) is used to treat the delay in ventricular contractions of the heart that occur in some people with advanced heart failure. A delay between the contraction of the right and left ventricles often occurs with heart failure, leading to biomechanically inefficient heart contractions and reduced cardiac output. Cardiac resynchronization therapy aims to address this problem through stimulation of multiple chambers of the heart in order to resynchronize contraction of the right and left ventricles.

Biomarkers, or biological markers, are substances that can be used as an indicator of a biological state. Biomarkers can include small molecules, proteins, nucleic acids, carbohydrates, lipids, and combinations thereof.

US 2011/0295084 A1 discloses an apparatus by which physiological sensor data can be combined with external biomarker assays to improve the sensitivity and specificity of heart failure detection.

M.D. Glick et al. (The Israel Medical Association Journal, June 2006, vol. 8, p. 391-394) teach that at baseline, coronary sinus levels of BNP, but not hsCRP, were significantly elevated compared to the PVB, wherein hsCRP levels or their change over two weeks did not predict all-cause mortality or hospitalizations.

F. Marin et al. (Thrombosis Research, 2011, vol. 128, p. 391-394) teach that a significant reduction in CRP levels is observed in post cardiac resynchronization therapy and that the biomarkers MMP-1 and MMP-2 showed significant changes at a time point four months post cardiac resynchronization therapy.

### SUMMARY OF THE INVENTION

Embodiments of the disclosure are related to systems and methods for utilizing biomarker panel data and related medical devices and methods, amongst other things. An embodiment can include a method of screening patients. The method can include quantifying levels of one or more of a panel of biomarkers in a biological sample of a patient. The method can further include analyzing the quantified levels. In some embodiments, the panel of biomarkers includes at least four selected from the group consisting of CRP, sST2, TIMP-1 or TIMP-2.

An embodiment can include a method of diagnosing a patient. The method can include quantifying levels of four or more of a panel of biomarkers in a biological sample of a patient. The method can further include diagnosing the patient based at least in part on the quantified levels. In some embodiments, the panel of biomarkers includes at least four selected from the group consisting of CRP, sST2, TIMP-1, TIMP-2.

An embodiment can include an implantable medical device. The implantable medical device can include a processor and can be configured to diagnose a patient based at least in part on quantified levels of one or more of a panel of biomarkers. In various embodiments, the panel of biomarkers including at least four selected from the group consisting of CRP, sST2, TIMP-1, TIMP-2.

This summary is an overview of some of the teachings of the present disclosure and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense. The scope of the present invention is defined by the appended claims and their legal equivalents. According to the invention, an implantable medical device having the features recited in claim 1 is provided and a method of screening patients having the features recited in claim 2 is provided.

### BRIEF DESCRIPTION OF THE DRAWINGS

The disclosure may be more completely understood in connection with the following drawings, in which:
FIG. 1 is a flow chart of some steps of methods in accordance with various embodiments herein.
FIG. 2 is a flow chart of some steps of methods in accordance with various embodiments herein.
FIG. 3 is a flow chart of some steps of a diagnosis method in accordance with various embodiments herein.
FIG. 4 is a schematic diagram of an exemplary system consistent with various embodiments herein.
FIG. 5 is a schematic diagram of various components of an external device in accordance with some embodiments of the disclosure.
FIG. 6 is a schematic diagram of various components of an implantable device in accordance with some embodiments of the disclosure.
FIG. 7 is a graph showing absolute change from baseline at 3 and 6 months for CRT responders versus non-responders for 12 biomarkers.

While the disclosure is susceptible to various modifications and alternative forms, specifics thereof have been shown by way of example and drawings, and will be described in detail. It should be understood, however, that the disclosure is not limited to the particular embodiments described. On the contrary, the intention is to cover modifications, equivalents, and alternatives falling within the spirit and scope of the disclosure.

### DETAILED DESCRIPTION

Embodiments herein include systems and methods for utilizing biomarker panel data in order to accomplish various tasks such as screening patients, diagnosing patients, and the like.

As used herein, the term "biomarker" shall include measurable substances that can be used as an indicator of a biological state including, without limitation, small molecules, proteins, nucleic acids, carbohydrates, lipids, and combinations thereof. It will be appreciated that reference to specific biomarkers herein shall include reference to precursors, proforms, isoforms, mature form variants, and degraded forms thereof including but not limited to metabolites thereof unless the context dictates otherwise.

It will be appreciated that many different types of biomarkers can be utilized in embodiments herein. Biomarkers used in various embodiments herein can include, but are not limited to adiponectin, adrenomedullin, midregion proadrenomedullin, angiotensin II, apelin, BNP, BNP1-32, NTproBNP1-76, caspase-3, connexin-43, copeptin, C-reactive protein (CRP), dihydropyridine receptor, epidermal growth factor (EGF), endoglin, endothelin, endothelin-1, big endothelin, eotaxin, fibroblast growth factor-2 (FGF-2), fibronectin, Flt-3 ligand, fractalkine, galectin 3 (Gal-3), G-CSF, GDF-15, GM-CSF, GRO, ICTP, IFNa2, IFN-gamma, IGF-1, IL-1, IL-1a, IL-1b, IL-1ra, IL-2, IL-3, IL-4, IL-5, IL-6, IL-7, IL-8, IL-9, IL-10, IL-12 (p40), IL-12 (p70), IL-13, IL-15, IL-17, IP-10, L-type calcium channel alpha subunit, MCP-1, MCP-3, MDC, MIP-1a, MIP-1b, miR-1, miR-21, miR-29, miR-30, miR-133, miR-208, miR-486, miR-760, MMP-1, MMP-2, MMP-3, MMP-7, MMP-8, MMP-9, MMP-12, MMP-13, MPO, osteopontin, PAI-I, PDGF-AA, PDGF-AB/BB, PIIINP, PINP, PRA-plasma rennin, RANTES, RNU 44, RNU 6B, sCD30, sCD40L, sEGFR, sE-selectin, sgp130, sICAM-I, sIL-1Rib, sIL-1RII, sIL-2Ra, sIL-4R, sIL-6R, sRAGE, ST2, sST2, sTNF RI, sTNF RII, sVCAM-I, sVEGFR1, sVEGFR2, sVEGFR3, TGFa, TGF bI, TGF bII, TGF bIII, TIMP-1, TIMP-2, TIMP-3, TIMP-4, TNFa, TNFb, TROPONIN-I, VEGF, ANP, NTproANP, midregion pro-ANP SERCA2a, ryanodine receptor, carboxy-terminal propeptide of procollagen type I (PICP), C-type natriuretic peptides (CNP, NTproCNP), aldosterone, epinephrine, arginine vasopressin (AVP), copeptin, urocortins I, II, III, Fas(APO-1), sFas, FasL, IL-18, oxidized low-density lipoproteins, myeloperoxidase, urine biopyrrins, urine and plasma isoprostanes, plasma malondialdehyde, carbonyl proteins, cardiac troponins I and T, myosin light-chain kinase I, heart fatty acid binding protein, creatine kinase MB fraction, ischemia modified albumin, osteoprotegerin, coenzyme Q10, sGp130, sTNFr-II, VAP-1, sVCAM-1, M-CSF, GM-CSF, and analogues thereof.

In some embodiments, biomarkers can include, but are not limited to, heart failure markers, inflammatory biomarkers, and/or remodeling biomarkers. In some embodiments, biomarkers can include, but are not limited to, biomarkers of inflammation, bioactive biomarkers, and/or proteolytic biomarkers.

It will be appreciated that a relatively diverse set of biomarkers can be relevant in terms of indicating inflammation. Specific biomarkers of inflammation in various embodiments herein can include, but are not limited to, CRP (C-reactive protein), SGP-130 (soluble glycoprotein 130), sIL-2R (soluble interleukin-2 receptor), sTNFR-II (soluble tumor necrosis factor receptor-II), IFN-γ (interferon gamma), sST2 (soluble suppressor of tumorigenicity-2), and fragments of any of these.

Bioactive biomarkers can include BNP (brain natriuretic peptide) and fragments thereof.

Proteolytic biomarkers can include MMPs and TIMPs. Matrix metalloproteinases (MMPs) are zinc-dependent endopeptidases. Synthesized as pro-enzymes, most MMPs are secreted before conversion to their active form. MMPs are capable of degrading extracellular matrix proteins in addition to processing a number of bioactive molecules. MMPs play an important role in breaking down components of the extracellular matrix (ECM). Specific MMPs for use as proteolytic biomarkers in various embodiments herein can include, but are not limited to, MMP-2; MMP-9; and fragments of any of these.

Tissue inhibitors of metalloproteinases (TIMPs) function by suppressing MMPs. Structurally, TIMPs contain two domains. The N-terminal domain binds to the active site of mature metalloproteases via a 1:1 non-covalent interaction, blocking access of substrates to the catalytic site. Specific TIMPs for use as proteolytic biomarkers in various embodiments herein can include, but are not limited to, TIMP-1, TIMP-2, TIMP-4; and fragments of any of these.

As used herein, the term "biomarker panel" shall refer to a set of biomarkers that can be used alone, together, or in subcombinations to indicate the status of a human subject with respect to a condition, status, or state of being of the human subject. The biomarkers within the panel of biomarkers can include those biomarkers discussed above. It will be appreciated that the specific identity of biomarkers within the panel and the number of distinct biomarkers within the panel can depend on the particular use to which the biomarker panel is put and the stringency that the results of panel must meet for the particular application.

In other embodiments, the panel includes at least four distinct biomarkers selected from CRP, sST2, TIMP-1 or TIMP-2.

Because the biomarkers within a particular biomarker panel can include a heterogeneous group in terms of their underlying biological function, it will be appreciated that each biomarker included within a panel may have a different normal biological concentration and therefore each biomarker included within a panel may have a different concentration or concentration threshold that is significant for indicating a particular biological status. By way of example, normal serum concentrations of CRP (C-reactive protein) are different from normal serum concentrations of IL-2, and therefore analyzing their concentrations for application in the context of a panel of biomarkers may require accounting for different inherent magnitudes in concentrations between the two.

In some embodiments each individual biomarker is assessed according to normal values (or reference normal values) for that biomarker in patient populations. In other words, each individual biomarker can be assessed as either being higher than normal, normal, or lower than normal. In some embodiments, the group is made up of healthy patients. In other embodiments, the group is patients with heart failure. In other embodiments, the group is patients with heart failure who have received CRT therapy devices. In still other embodiments, the group is made up of patients with heart failure who are responders to CRT therapy. In some embodiments, the group is made up of patients with heart failure who are not responders to CRT therapy.

Analysis of the biomarker panel can result in a positive result, a negative result, or an indeterminate result. In some embodiments, a positive biomarker panel result can be defined based on all of the biomarkers within the panel exceeding a threshold value. In other embodiments, a positive biomarker panel result can be defined based on a majority of the biomarkers within the panel exceeding a threshold value. In some embodiments, the threshold value can be a unique quantitative concentration for that particular biomarker. In other embodiments, the threshold value can be more qualitatively assessed as either normal or abnormal; or higher than normal, normal, or lower than normal.

Various embodiments herein can include quantifying levels of one or more of a panel of biomarkers in a biological sample of a patient. In some embodiments, quantifying levels can be conducted *in vitro.* It will be appreciated that many different techniques exist for quantifying levels of biomarkers, depending of course on the nature of the specific biomarker. For example, in the context of identifying proteins or fragments thereof methods used can include, but are not limited to, ELISA (enzyme linked immunosorbent assay), western blotting, other types of electrophoretic analytic assays, immunohistochemical staining, affinity chromatography, mass spectrometry, and the like. In some embodiments, quantifying levels of one or more biomarkers can include quantifying levels of other compounds that would be indicative of those levels. By way of example, in the context of quantifying levels of a particular protein, detection of mRNA coding for the protein or precursors thereof can be used in order to quantify such levels. As such, in various embodiments, techniques can be used including northern blotting, RT-PCR (polymerase chain reaction), serial analysis of gene expression (SAGE), microarray assays, and the like. In the context of quantifying levels of particular proteins, other compounds that are indicative of those levels can include precursors, proforms, isoforms, mature form variants, and degraded forms including but not limited to metabolites thereof. It will be appreciated, however, that many other techniques exist that can be used to detect and/or quantify levels of biomarkers.

In some embodiments, other types of data can be used in conjunction with biomarker data for methods herein. By way of example, data can include but is not limited to, data regarding patient gender, ischemic or non-ischemic origin of heart failure, the presence or absence of left bundle branch block, QRS width (such as QRS width greater than or equal to 150 milliseconds), information on prior hospitalizations, left ventricular end-diastolic volume (LVEDV) (such as LVEDV greater than 125 mL/m², and left atrial volume (such as left atrial volume less than 40 mL/m²). Use of such other types of data can include their use as binary variable or continuous variables where appropriate.

In some embodiments, the data used can include ST2, CRP, TIMP1, TIMP2, and one, two, three, or more of LVESV at baseline, age at baseline, sex, systolic blood pressure at baseline, QRS duration at baseline, PR interval at baseline, ischemic status at baseline, and ACE (angiotensin-converting enzyme inhibitors) / ARB (angiotensin II receptor blockers) use at baseline.

In some embodiments, the data used can include CRP, BNP, and one, two, three, or more of LVESV at baseline, age at baseline, sex, systolic blood pressure at baseline, presence of left bundle branch block at baseline, renal disease at baseline, ischemic status at baseline, and beta blocker use at baseline.

In some embodiments, the data used can include CRP, BNP, and one, two, three, or more of LVESV at baseline, age at baseline, sex, systolic blood pressure at baseline, QRS duration at baseline, PR interval at baseline, ischemic status at baseline, beta blocker use at baseline, and ACE/ARB use at baseline.

In some embodiments, the data used can include BNP, CRP, TNFR-II, and one, two, three, or more of LVESV at baseline, age at baseline, sex, systolic blood pressure at baseline, QRS duration at baseline, presence of left bundle branch block at baseline, ischemic status at baseline, and renal disease at baseline.

It will be appreciated that various samples can be used as the biological sample for testing in accordance with various embodiments herein. By way of example, samples can include, but are not limited to fluid samples (such as plasma, blood, blood serum, interstitial fluid, bodily filtrates, pleural fluid, lymph fluids, cerebrospinal fluid, mucus, peritoneal fluid, saliva, sweat, tears, urine, as well as other secretions, and the like) as well as tissue samples. In various embodiments, the method can further include the step of taking a biological sample of a patient.

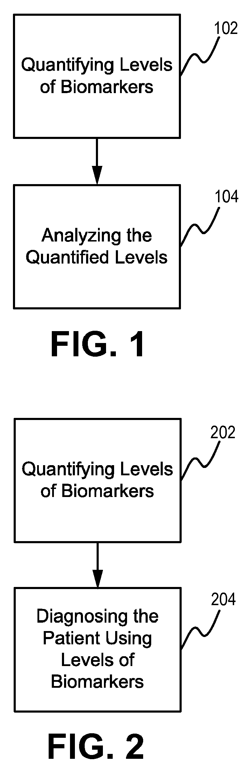

Referring now to FIG. 1, in some embodiments, methods herein can include quantifying levels of one or more of a panel of biomarkers 102 in a biological sample of a patient. Quantification of the levels of the biomarkers in the panel can be performed in various ways, such as through the use of various techniques described above. In some embodiments, the quantification is an absolute value of the biomarker or panel of biomarkers. In some embodiments, the quantification is a relative value with respect to values for other markers, genomic or proteomic targets, or reference values. In still other embodiments, the quantification may reflect a change in levels over a time period (such as the time period between follow-up visits to a clinician or the time period between implant of a device and a follow-up visit to a clinician). In some embodiment, data regarding biomarker levels for a particular patient can be tracked over an extended period of time including measurements at a plurality (e.g., 2-100 or more) of time points and an overall profile of changes can be determined.

Methods can also include analyzing the quantified levels 104. For example, methods can include analyzing the quantified levels in order to screen patients. For example, patients can be screened for heart failure, other heart conditions, and/or for candidacy as a recipient of a CRT device, or the like. In some embodiments, patients can be screened for the presence of heart failure along with a co-morbidity or secondary pathology. In some embodiments, heart failure along with a co-morbidity or secondary pathology can be identified in patients. By way of example, patients can be screened for (or the following can be identified) heart failure and renal disease, heart failure and diabetes, heart failure and cancer, heart failure and arthritis, or the like. In some embodiments, patients can be screened for heart failure and the presence of a circumstance that may prevent them from reaching or maintaining an optimal state such as heart failure and sub-optimal pharmacotherapy or heart failure and improper diet (such as too much sodium).

Analyzing the quantified levels can include various operations. In some embodiments, analyzing the quantified levels includes comparing quantified levels to reference normal levels to screen patients.

The reference normal levels can be determined in various ways. In some embodiments, the reference normal levels are determined based on a group of patients. In some embodiments, the group is made up of healthy patients. In other embodiments, the group is patients with heart failure. In other embodiments, the group is patients with heart failure who have received CRT therapy devices. In still other embodiments, the group is made up of patients with heart failure who are responders to CRT therapy. In some embodiments, the group is made up of patients with heart failure who are not responders to CRT therapy.

In some embodiments, analyzing the quantified levels includes calculating an aggregate score that represents the deviations of quantified levels from reference normal levels for the panel of biomarkers. Positive or negative deviations from reference normal levels can be relevant to screening patients. In some embodiments, the specific degree of positive or negative deviations from reference normal levels can be indicative. In some embodiments, non-deviation from reference normal levels can be indicative. In some embodiments, analyzing the quantified levels comprises comparing quantified levels to threshold levels to screen patients.

In some embodiments, methods can further include selecting a patient. In some embodiments, the patient can be selected on the basis of symptoms. For example, selecting the patient can be done on the basis of heart failure symptoms. In some embodiments, the symptoms can include subjective symptoms. In other embodiments, the symptoms can include objective symptoms. In still other embodiments, the symptoms can include combinations of subjective and objective symptoms. In some embodiments, selecting the patient can be based on what the patient is indicated for. By way of example, selecting the patient can be based on the patient being indicated for CRT therapy. In some embodiments, selecting the patient can be based on the patient suffering from heart failure, but not being indicated for CRT therapy.

In some embodiments, methods can include placing the patient into a category. For example, some embodiments are directed to a method of screening patients and/or diagnosing patients and using the quantified levels to place the patient into one of a set of categories. Categories can include, but are not limited to, one or more of: normal, at-risk for heart failure, at-risk for rapidly progressing heart failure, suffering from heart failure, suffering from rapidly progressing heart failure (such as rapidly progressing cell/chamber remodeling), likely to benefit from treatment, unlikely to benefit from treatment, and the like. In some embodiments, the set of categories can include at least two categories. In some embodiments, the set of categories can include at least three categories. In some embodiments, the set of categories can include at least four categories. In some embodiments, the set of categories can include at least five categories.

In some embodiments, placement into a category can be based on the difference between quantified levels of one or more of the panel of biomarkers and reference normal levels for one or more of the panel of biomarkers. In some embodiments, different biomarkers receive equal weighting. In other embodiments, different biomarkers received different weighting.

In some embodiments, a method of diagnosing a patient is included herein. Referring now to FIG. 2, the method can include quantifying levels of one or more of a panel of biomarkers in a biological sample of a patient 202. The method can further include diagnosing the patient based at least in part on the quantified levels 204. In some embodiments, methods of diagnosing a patient can include of selecting a patient exhibiting symptoms of heart failure. Symptoms of heart failure can include, but are not limited to, exercise intolerance, breathlessness, cardiomegaly, pulmonary edema, and evidence of decompensation events.

It will be appreciated that diagnosing can proceed in various ways. In some embodiments, diagnosing the patient can include determining if the patient has a unique heart failure etiology. In some embodiments, diagnosing the patient includes comparing the quantified levels of biomarkers to reference levels indicative of heart failure.

In some embodiments, diagnosing the patient includes comparing the quantified levels of biomarkers to reference levels indicative of heart failure in conjunction with analyzing other types of data. Other types of data can include traditional data used for the diagnosis of heart failure such as echocardiogram data, other types of imaging data (including but not limited to MRI, CT, and the like), electrocardiogram data (including but not limited to QRS widths, indications of conduction problems such as right bundle branch block and left bundle branch block, and other electrocardiogram data), chronotropic competence, endomyocardial biopsy data, data from implanted devices (such as heart rate variablility, activity, percent pacing, and the like), cardiac function data (such as left ventricle ejection fraction (LVEF), cardiac output, and the like), structure information (such as left ventricular end-systolic volume (LVESV), left ventricular end-diastolic volume (LVEDV), left ventricle (LV) diameter, and the like), clinical status, clinical history, medications taken, performance tests (such as 6 minute hall walk, VO₂ max), ischemia, and symptoms such as exercise intolerance, breathlessness, cardiomegaly, pulmonary edema, evidence of decompensation events, and the like.

In still other embodiments, diagnosis can rely upon data regarding changes in levels over a time period (such as the time period between follow-up visits to a clinician or the time period between implant of a device and a follow-up visit to a clinician). In some embodiments, the trends in changes shown by the biomarker panel can take on significance for diagnosis that exceeds that of the absolute values of individual components of the biomarker panel. In some embodiments, data regarding biomarker levels for a particular patient as tracked over an extended period of time including measurements at a plurality of time points can be used to reach a diagnosis.

In some embodiments, diagnosing the patient can include using quantified levels of biomarkers to confirm a diagnosis of heart failure made according to traditional diagnosis algorithms. In some embodiments, diagnosing the patient can include using quantified levels of biomarkers to determine whether the patient has heart failure when a patient exhibits some symptoms of heart failure but the diagnosis is indeterminate.

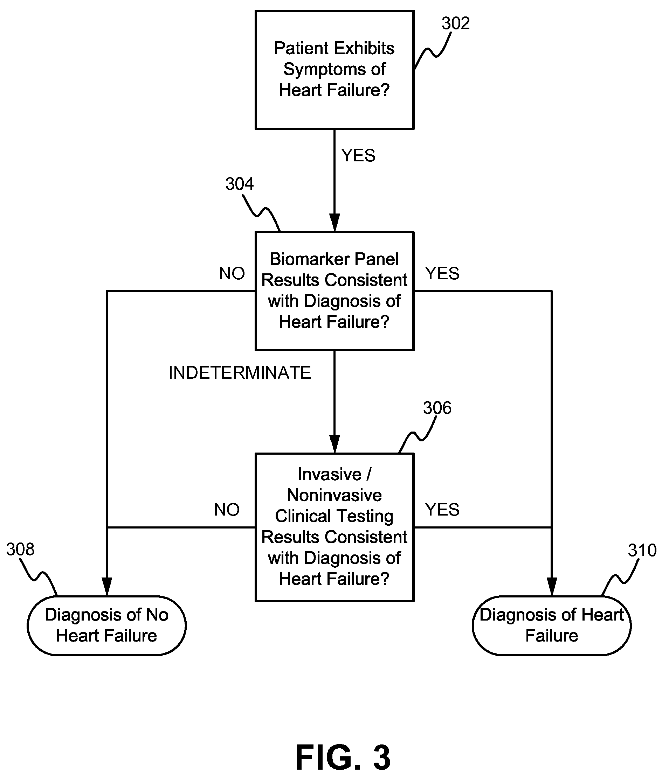

In still other embodiments, diagnosing the patient can include using quantified levels of biomarkers to try to reach diagnosis of heart failure before undertaking costly and invasive techniques to try to diagnose the condition. Referring now to FIG. 3, it can be determined whether a patient exhibits symptoms of heart failure 302. If the patient is exhibiting symptoms of heart failure 302, then in another operation it can be determined if the biomarker panel yields results consistent with a diagnosis of heart failure 304. If the answer is yes, then a diagnosis of heart failure can be made 310. If the answer is no, then a diagnosis of no heart failure can be made 308. If the results of the biomarker panel analysis are indeterminate, then further steps such as invasive and/or noninvasive clinical testing can be performed in order determine if the results are consistent with a diagnosis of heart failure 306. If the further testing is positive, then a diagnosis of heart failure can be made 310. If the further testing is negative, then a diagnosis of no heart failure can be made 308.

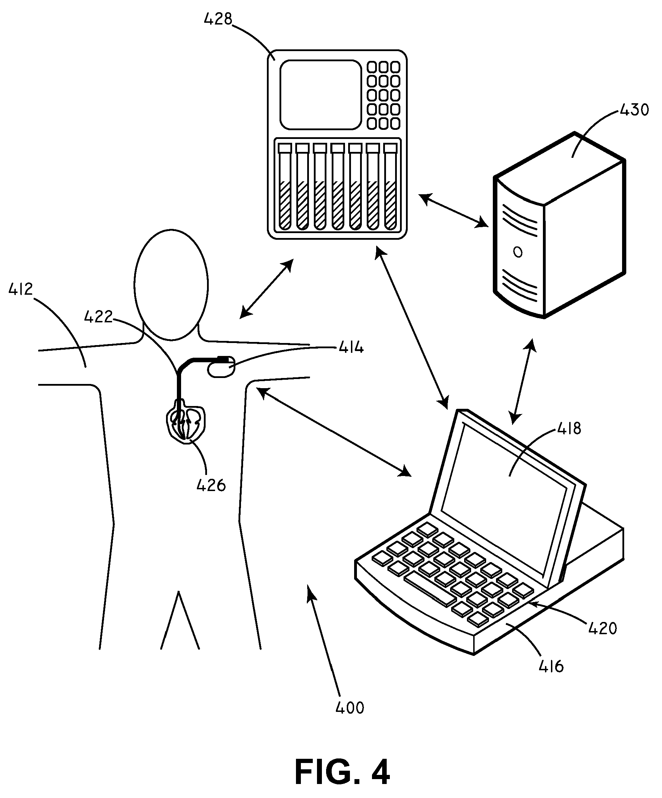

Some embodiments can include systems and/or devices. However, it will be appreciated that embodiments related to biomarkers herein are not limited to implementation through devices or systems. FIG. 4 is a schematic view of some system 400 components, consistent with various embodiments herein. Figure 4 schematically illustrates a biomarker diagnostic test being run and the values being communicated with a server as well as a device programmer (external medical device) and an implantable medical device. In some embodiments, the programmer can communicate with the device and based on the biomarker values reprogram the device settings as appropriate. Some embodiments can include all of the components shown in FIG. 4, while other embodiments include only some of the components. Data can pass from various specific components to other components within the system as indicated by the arrows. However, it will be appreciated that communication is not restricted to such pathways and that communication of data between devices can take place in various ways, automatically or through manual data input processes. The system 400 can include an implantable medical device 414. Methods described herein, or portions thereof, can be executed by the implantable medical device 414. The implantable medical device 414 can be disposed within a patient 412. The implantable medical device 414 can be of various types such as, for example, a pacemaker, a cardioverter-defibrillator, a cardiac resynchronization device, or the like. In some embodiments, the implantable medical device 414 is specifically a cardiac resynchronization device. One example of an implantable medical device is disclosed in commonly assigned U.S. Pat. No. 4,562,841. In some embodiments, the implantable medical device 414 can include one or more leads 422 disposed in or near the patient's heart 426. The leads can include a plurality of electrodes, such as ring and/or tip electrodes. In some embodiments, leads 422 can be positioned in both the left ventricle and the right ventricle of the heart.

The implantable medical device 414 can be in communication with an external medical device 416. Methods described herein, or portions thereof, can be executed by the external medical device 416. In some embodiments, communication between the implantable medical device 414 and the external medical device 416 can be via inductive communication through a wand 410 held on the outside of the patient 412 near the implantable medical device 414. However, in other embodiments, communication can be carried out via radiofrequency transmission, acoustically, or the like.

The external medical device 416 can include a video output device, such as a display screen 418 for displaying video output. In some embodiments, the external medical device 416 can be configured to process the gathered data. The external medical device 416 can also include a user input device 420, such as keys. The external medical system 416 can be for example, a programmer/recorder/monitor device, a computer, an advanced patient management system, or a personal digital assistant (PDA). Exemplary programmer/recorder/monitor devices include the Model 3120 Programmer, available from Boston Scientific Corporation, Natick, MA.

In some embodiments the implantable medical device 414 can include one or more implantable sensors in order to gather data regarding the patient 412. By way of example, the medical device 414 can include an implantable sensor in order to sense concentrations of a predictive marker. In some embodiments, the implantable sensor can sense concentrations of CRP *in vivo.* Exemplary implantable sensors are described in U.S. Publ. Pat. Appl. No. 2007/0270675.

In some embodiments, concentrations of biomarkers can be measured using an *in vitro* assay. By way of example, in some embodiments, an external assay device 428 can be used to measure concentrations of a biomarker such as CRP. The patient 412 can provide an assayable quantity of blood or other bodily fluid to the external assay device 428 and the device can measure the amount of the biomarker present and then report back regarding concentration data either directly to the implantable medical device 414 or through the external medical device 416.

In some embodiments, the system 400 can include a server 430. The server 430 can be located remotely from other components of the system 400 but can be in communication such as through a network, such as a packet switched network. The server 430 can be in communication with one or more of the implantable medical device 414, the external medical device 416, and the external assay device 428, when such components are part of the system 400. The server 430 can include standard server components such as processor, memory, input/output interfaces, and the like. In some embodiments, the server 430 can further be in communication with a database (not shown). Methods described herein, or portions thereof, can be executed by the server 430.

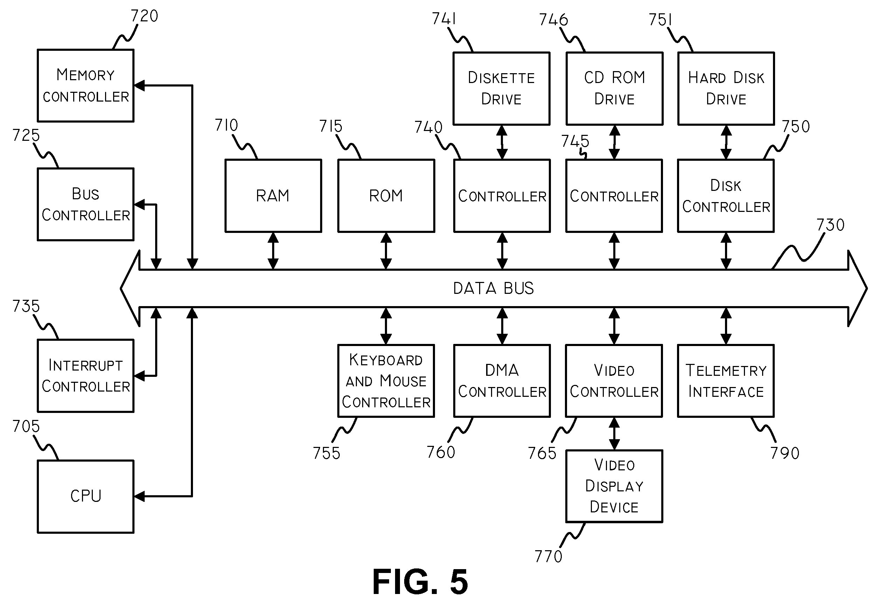

In some embodiments, one or more operations, methods described herein, or portions thereof can be executed by an external device. Exemplary external devices, such as programmer/recorder/monitors, can include components common to many computing devices. Referring now to FIG. 5, a diagram of various components is shown in accordance with some embodiments. The external system includes a central processing unit (CPU) 705 or processor, which may include a conventional microprocessor, random access memory (RAM) 710 for temporary storage of information, and read only memory (ROM) 715 for permanent storage of information. A memory controller 720 is provided for controlling system RAM 710. A bus controller 725 is provided for controlling data bus 730, and an interrupt controller 735 is used for receiving and processing various interrupt signals from the other system components.

In some embodiments mass storage can be provided by diskette drive 741, which is connected to bus 730 by controller 740, CD-ROM drive 746, which is connected to bus 730 by controller 745, and hard disk drive 751, which is connected to bus 730 by controller 750. User input to the programmer system may be provided by a number of devices. For example, a keyboard and mouse can connected to bus 730 by keyboard and mouse controller 755. DMA controller 760 is provided for performing direct memory access to system RAM 710. A visual display is generated by a video controller 765 or video output, which controls video display 770. The external system can also include a telemetry interface 790 or telemetry circuit which allows the external system to interface and exchange data with an implantable medical device or an external device. In some embodiments, the external system can include a network interface. The external programmer device can be configured to receive data regarding levels of one or more of a panel of biomarkers in a biological sample of a patient. This description of elements is only provided by way of example and it will be appreciated that some embodiments may lack various elements illustrated in FIG. 5. For example, some embodiments of external devices may lack a diskette drive 741.

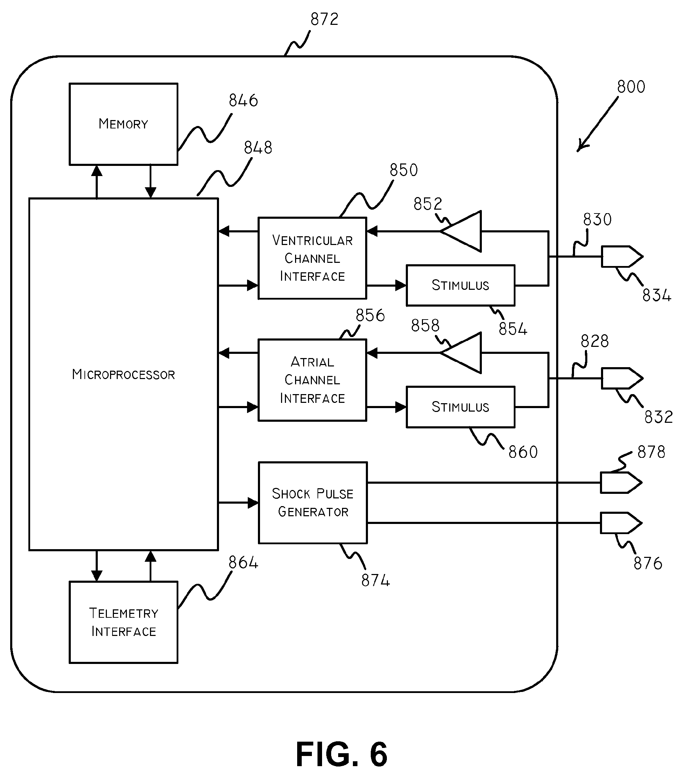

In some embodiments, one or more operations, methods described herein, or portions thereof can be executed by an implantable medical device. Referring now to FIG. 6, some components of an exemplary implantable device 800 are schematically illustrated. The implantable medical device 800 can include a controller module 872 coupled to one or more stimulation leads 830 and 828. The controller module 872 can include a microprocessor 848 (or processor) that communicates with a memory circuit (module) 846 via a bidirectional data bus. The memory circuit 846 can include ROM or RAM for program storage and ROM (such as EEPROM) or RAM for data storage. The controller module 872 can be configured to execute various operations such as processing of signals and execution of methods or portions thereof as described herein. For example, the controller module can be configured to process data regarding the levels of one or more of a panel of biomarkers. A telemetry interface 864 or communication circuit is also provided for communicating with an external unit, such as a programmer device or a patient management system. The communication circuit can be configured to receive data regarding levels of one or more of the panel of biomarkers.

The controller module 872 can include one or more ventricular sensing and pacing channels including sensing amplifier 852, output circuit 854, and a ventricular channel interface 850 which communicates bidirectionally with a port of microprocessor 848. Further, in some embodiments, additional elements may be included. The ventricular sensing and pacing channel can be in communication with stimulation lead 830 and electrode 834.

The controller module 872 can include one or more atrial sensing and pacing channels including sensing amplifier 858, output circuit 860, and an atrial channel interface 856 which communicates bidirectionally with a port of microprocessor 848. The atrial sensing and pacing channel can be in communication with stimulation lead 828 and electrode 832. For each channel, the same lead and electrode can be used for both sensing and pacing. The channel interfaces 850 and 856 can include analog-to-digital converters for digitizing sensing signal inputs from the sensing amplifiers and registers which can be written to by the microprocessor in order to output pulses, change the pacing pulse amplitude, and adjust the gain and threshold values for the sensing amplifiers. It will be appreciated that in some embodiments some of the elements of the controller module 872 shown in FIG. 6 may be omitted.

A shock pulse generator 874 can also be interfaced to the microprocessor for delivering defibrillation shocks to the heart via a separate pair of electrodes 876, 878. In some embodiments, electrodes 876 and 878 can be disposed along stimulation lead 830 and stimulation lead 828 respectively. In some embodiments, one or more of these components may be omitted. By way of example, if the implantable medical device is a pacemaker, then it may not include a shock pulse generator 874. Similarly, depending on the type of the device and its configuration, it may have a greater or lesser number of electrodes and channels.

It should be noted that, as used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

It should also be noted that, as used in this specification and the appended claims, the phrase "configured" describes a system, apparatus, or other structure that is constructed or configured to perform a particular task or adopt a particular configuration. The phrase "configured" can be used interchangeably with other similar phrases such as "arranged", "arranged and configured", "constructed and arranged", "constructed", "manufactured and arranged", and the like.

One of ordinary skill in the art will understand that the modules, circuitry, and methods shown and described herein with regard to various embodiments can be implemented using software, hardware, and combinations of software and hardware. As such, the illustrated and/or described modules and circuitry are intended to encompass software implementations, hardware implementations, and software and hardware implementations.

All publications and patent applications in this specification are indicative of the level of ordinary skill in the art to which this disclosure pertains.

This application is intended to cover adaptations or variations of the present subject matter. It is to be understood that the above description is intended to be illustrative, and not restrictive. The scope of the present subject matter should be determined with reference to the appended claims, along with the full scope of equivalents to which such claims are entitled.

### Examples

### Example 1: Biomarkers Relevant to Heart Failure

Plasma was collected at baseline (during CRT evaluation, but prior to CRT implant), at 3 and 6 months post-CRT placement in a prospective fashion, from patients with heart failure enrolled in a trial (n=764). See Ellenbogen et al., Primary results from the Smart Delay determined AV optimization: a comparison to other AV delay methods used in cardiac resynchronization therapy (SMART-AV) trial. Circulation 2010;122:2660-2668. The trial established a pre-specified endpoint of a reduction in LV end-systolic volume of 15 mL at 6 months post-CRT as a functional response and this definition was utilized in this biomarker profiling study. The initial goal of the trial was to compare 3 different modes of CRT atrioventricular (A-V) delay, which following randomization demonstrated relative equivalency for the primary endpoint, and therefore these patients were pooled for the present analysis.

Specifically, peripheral venous blood samples were collected from patients with heart failure who meet standard indications for CRT in the United States and Europe. Samples were collected by standard venipuncture in two (2) ethylenediamine tetraacetic acid (EDTA, 18 mg purple top) tubes (BD Vacutainer, 366643). Centrifugation was done at room temperature for 10 minutes at high speed (recommended to be ≥ 3,000 revolutions per minute (RPM) or ≥ 1300 g (relative centrifugal force=RCF=g)). The plasma from each blood sample tube was decanted into two separate and properly labeled polypropylene tubes. The plasma was kept on ice until it is transferred to a storage freezer. Plasma samples were frozen as soon as possible following centrifugation. The plasma sample were then stored in a -20°C freezer until shipment. Samples were shipped to the analysis facility within 72 hours from enrolling centers.

90 candidate plasma proteins which spanned functional domains such as signaling, inflammation, myocyte/matrix structure for extraction efficiency, detection thresholds, and variability in referent non-device patients (n=25). This was followed by a validation phase of 18 candidate biomarkers in a second subset of the SMART-AV patient samples (n=25/25; responder/non-responder). For example, IFN-gamma was analyzed using an array (Millipore Cytokine Panel); sIL-2RA, and sTNF-RII were analyzed using an array (Millipore Cytokine Receptor Panel), sVCAM-1 was analyzed using an array (Millipore HCVD1 Panel); MMP-2 (LMP902; RnD Systems) and MMP-9 (LMP911; RnD Systems) were analyzed using an MMP Multiplex Array Panel (RnD Systems - Cat# LMP000); TIMP-1 was analyzed using TIMP Array Panel (Cat# LKT003; RnD Systems); CRP was analyzed using an ELISA assay (Cat# DCRP00; RnD Systems), BNP was analyzed using an ELISA assay (Cat# 04-BI-20852, ALPCO Immunoassays); ST2 was analyzed using an ELISA assay (Cat# DST200; RnD Systems).

Using logistic regression and area under the curve (AUC), a final set of 12 biomarkers were analyzed by internally validated, robotic assisted, high sensitivity multiplex suspension array on the entire prospectively collected sample set (>2200 samples) where at 6 months, through independent review, 338 patients were defined as responders and 376 defined as non-responders.

The 12 biomarkers represented several domains: inflammatory (C-reactive protein: CRP, soluble glycoprotein 130: SGP-130, soluble interleukin-2 receptor: sIL-2R, soluble tumor necrosis factor receptor-II: sTNFR-II, interferon gamma: IFNg), bioactive (brain natriuretic peptide: BNP, soluble suppressor of tumorgenicity-2: sST2), and proteolytic (matrix metalloproteinase -2 and -9: MMP-2/-9, tissue inhibitors of MMP: TIMP-1, -2, -4). Significant differences in absolute values for several of these prospectively collected biomarkers occurred at baseline between those defined as responders and non-responders at 6 months, such as sST2 and sTNFR-II (31219±980 vs 35435±1120, and 8839±497 vs 9145±281 pg/mL, p<0.05, respectively).

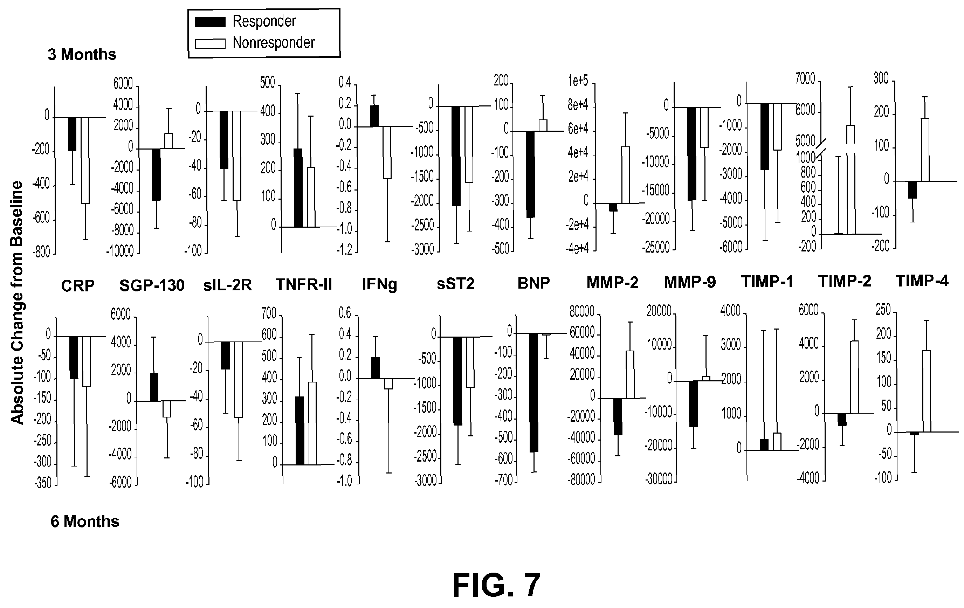

More importantly, including the baseline measurements of all 12 biomarkers in a multivariable regression model yielded a significant AUC (0.60, p<0.05) for the prediction of CRT response at 6 months. In addition, a significant and differential biomarker profiles occurred as a function of time, between responders and non-responders to CRT (Figure). For example, at 3 months post-CRT significant and directional changes in BNP, MMP-2, TIMP-2 and TIMP-4 occurred between responders/non-responders (p<0.05), and a multivariable analysis of all 12 biomarkers at 3 months predicted 6 month response (AUC=0.64, p<0.05). FIG. 7 is a graph showing absolute change from baseline at 3 and 6 months for CRT responders versus non-responders for the 12 selected biomarkers. However, it will be appreciated that relative changes can also be considered in various embodiments.

The new and unique findings from this prospectively designed plasma profiling study were that a specific biomarker panel can successfully predict CRT response in patients with HF indicated for a device (NYHA Class III/IV, EF **≤** 35%, QRS duration > 120 ms), as well as be utilized to monitor functional response following CRT. It will be appreciated, however, that embodiments herein are not limited to patients with that specific indication. Thus, plasma biomarker profiling can provide prognostic utility when evaluating patients with HF for CRT as well as an adjunctive diagnostic tool for monitoring functional response in the early period following CRT placement.

Multivariate logistic regression modeling was performed to assess positive responders (defined as change in LVESV (left ventricular end-systolic volume) from baseline ≤ -15 ml) versus neutral/negative responders (defined as death within first 6 months or LVESV change from baseline > -15ml). The following biomarker and clinical covariates included in the final multivariate model were selected using 10-fold cross validation methodology: ST2 (continuous variable), CRP (continuous variable), TIMP1 (continuous variable), TIMP2 (continuous variable), LVESV at baseline (continuous variable), age at baseline (continuous variable), sex (binary variable), systolic blood pressure at baseline (continuous variable), QRS duration at baseline (continuous variable), PR interval at baseline (continuous variable), ischemic status at baseline (binary variable), ACE (angiotensin-converting enzyme inhibitors) / ARB (angiotensin II receptor blockers) use at baseline (binary variable). Area under the curve (AUC) obtained via cross-validation for the final set of covariates equaled 0.731; AUC obtained by fitting the model on the entire dataset equaled 0.754.

Multivariate logistic regression modeling was performed to assess negative responders (defined as death within first 6 months or LVESV change from baseline ≥ 20ml) versus positive/neutral responders (defined as LVESV change from baseline < 20ml). The following biomarker and clinical covariates included in the final multivariate model were selected using 10-fold cross validation methodology: CRP (continuous variable), BNP (continuous variable), LVESV at baseline (continuous variable), age at baseline (continuous variable), sex (binary variable), systolic blood pressure at baseline (continuous variable), presence of left bundle branch block at baseline (binary variable), renal disease at baseline (binary variable), ischemic status at baseline (binary variable), beta blocker use at baseline (binary variable). Area under the curve (AUC) obtained via cross-validation for the final set of covariates equaled 0.676; AUC obtained by fitting the model on the entire dataset equaled 0.715.

Multivariate logistic regression modeling was performed to assess positive responders (defined as change in LVESV from baseline ≤ -15 ml) versus negative responders (defined as death within first 6 months or LVESV change from baseline ≥ 20ml). The following biomarker and clinical covariates included in the final multivariate model were selected using 10-fold cross validation methodology: CRP (continuous variable), BNP (continuous variable), LVESV at baseline (continuous variable), age at baseline (continuous variable), sex (binary variable), systolic blood pressure at baseline (continuous variable), QRS duration at baseline (continuous variable), PR interval at baseline (continuous variable), ischemic status at baseline (binary variable), beta blocker use at baseline (binary variable), ACE/ARB use at baseline (binary variable). Area under the curve (AUC) obtained via cross-validation for the final set of covariates equaled 0.739; AUC obtained by fitting the model on the entire dataset equaled 0.765.

Multivariate cumulative logistic regression modeling was performed to assess response as a three level outcome: positive responders (defined as change in LVESV from baseline ≤ -15 ml), neutral responders (defined as LVESV change from baseline > -15ml and < 20ml), and negative responders (defined as death within first 6 months or LVESV change from baseline ≥ 20ml). The following biomarker and clinical covariates included in the final multivariate model were selected using 10-fold cross validation methodology: BNP (continuous variable), CRP (continuous variable), TNFR-II (continuous variable), LVESV at baseline (continuous variable), age at baseline (continuous variable), sex (binary variable), systolic blood pressure at baseline (continuous variable), QRS duration at baseline (continuous variable), presence of left bundle branch block at baseline (binary variable), ischemic status at baseline (binary variable), renal disease at baseline (binary variable). Area under the curve (AUC) obtained via cross-validation for the final set of covariates equaled 0.681; AUC obtained by fitting the model on the entire dataset equaled 0.696.

## Claims

1. An implantable medical device, wherein the implantable medical device is a pacemaker, a cardioverter-defibrillator or a cardiac resynchronisation device, comprising:
a controller module coupled to one or more stimulation leads, wherein
the controller module comprises a processor, a memory circuit for communicating with the processor via a bidirectional data bus, and a telemetry interface for communicating with an external unit;
the implantable medical device configured to determine if a patient has a particular heart failure etiology, based at least in part on quantified levels of one panel of biomarkers, wherein the device is configured to compare the quantified levels to i) reference levels indicative of heart failure and ii) reference normal levels; and
wherein the device is configured to analyze echocardiogram data;
the panel of biomarkers including at least CRP, sST2, TIMP-1 and TIMP-2.

2. A method of screening patients:
quantifying levels of one panel of biomarkers in a biological sample of a patient, wherein quantifying levels is conducted in vitro; and
analyzing the quantified levels, wherein analyzing the quantified levels includes comparing quantified levels to reference normal levels and
wherein analyzing the quantified levels includes using the quantified levels to place the patient into one of a set of categories relating to risk of heart failure progression;
wherein the panel of biomarkers includes at least CRP, sST2, TIMP-1 and TIMP-2,
wherein analyzing the quantified levels includes determining whether the patient is at risk for
i) experiencing heart failure decompensation; and/or
ii) rapid decline in clinical symptoms of heart failure; and/or
iii) adverse ventricular remodeling; and/or
iv) arrhythmias.

3. The method of claim 2, the biological sample comprising plasma.

## Patentansprüche

1. Implantierbare medizinische Vorrichtung, die ein Schrittmacher, ein Cardioverter-Defibrillator oder eine Herzresynchronisationsvorrichtung ist, und welche aufweist:
ein Steuermodul, das mit einer oder mehreren Stimulationsleitungen gekoppelt ist, wobei das Steuermodul einen Prozessor, eine Speicherschaltung zum Kommunizieren mit dem Prozessor über einen bidirektionalen Datenbus und eine Telemetrie-Schnittstelle zum Kommunizieren mit einer externen Einheit aufweist;
wobei die implantierbare medizinische Vorrichtung konfiguriert ist zum Bestimmen, ob ein Patient eine bestimmte Herzversagensätiologie hat, zumindest teilweise basierend auf der Grundlage von quantifizierten Pegeln eines Feldes von Biomarkern,
wobei die Vorrichtung konfiguriert ist zum Vergleichen der quantifizierten Pegel mit i) Bezugspegeln, die ein Herzversagen anzeigen, und ii) Bezugsnormalpegeln; und
wobei die Vorrichtung konfiguriert ist zum Analysieren von Echocardiogrammdaten;
wobei das Feld von Biomarkern zumindest CRP, sST2, TIMP-1 und TIMP-2 enthält.

2. Verfahren zum Screenen von Patienten:
Quantifizieren von Pegeln eines Feldes von Biomarkern in einer biologischen Probe eines Patienten, wobei das Quantifizieren von Pegeln in vitro durchgeführt wird; und
Analysieren der quantifizierten Pegel, wobei das Analysieren der quantifizierten Pegel das Vergleichen quantifizierter Pegel mit Bezugsnormalpegeln umfasst, und wobei das Analysieren der quantifizierten Pegel die Verwendung der quantifizierten Pegel zum Einordnen des Patienten in eine von einem Satz von Kategorien, die sich auf die Gefahr des Fortschreitens von Herzversagen beziehen, umfasst;
wobei das Feld von Biomarkern zumindest CRP, sST2, TIMP-1 und TIMP-2 enthält,
wobei das Analysieren der quantifizierten Pegel das Bestimmen enthält, ob der Patient gefährdet ist für
i) das Erleiden einer Herzversagens-Dekompensation; und/oder
ii) rasche Abnahme von klinischen Symptomen von Herzversagen; und/oder
iii) nachteilige ventrikuläre Umbildung; und/oder
iv) Arrhythmien.

3. Verfahren nach Anspruch 2, bei dem die biologische Probe Plasma aufweist.

## Revendications

1. Dispositif médical implantable, dans lequel le dispositif médical implantable est un stimulateur cardiaque, un dispositif de cardioversion-défibrillateur ou un dispositif de resynchronisation cardiaque, comprenant :
un module de contrôleur couplé à une ou plusieurs connexion(s) de stimulation, dans lequel le module de contrôleur comprend un processeur, un circuit de mémoire pour communiquer avec le processeur via un bus de données bidirectionnel et une interface de télémétrie pour communiquer avec une unité externe,
le dispositif médical implantable étant configuré de manière à déterminer si un patient présente une étiologie de défaillance cardiaque particulière, sur la base au moins en partie de niveaux quantifiés d'un panel de biomarqueurs, dans lequel le dispositif est configuré de manière à comparer les niveaux quantifiés à i) des niveaux de référence indicatifs d'une défaillance cardiaque et à ii) des niveaux normaux de référence ; et dans lequel :
le dispositif est configuré de manière à analyser des données d'électrocardiogramme,
le panel de biomarqueurs incluant au moins CRP, sST2, TIMP-1 et TIMP-2.

2. Procédé de sélection de patients :
la quantification de niveaux d'un panel de biomarqueurs dans un échantillon biologique d'un patient, dans lequel la quantification des niveaux est mise en oeuvre in vitro ; et
l'analyse des niveaux quantifiés, dans lequel l'analyse des niveaux quantifiés inclut la comparaison de niveaux quantifiés à des niveaux de référence normaux et dans lequel l'analyse des niveaux quantifiés inclut l'utilisation des niveaux quantifiés de manière à placer le patient dans une catégorie d'un jeu de catégories se rapportant à un risque de progression de défaillance cardiaque ; dans lequel :
le panel de biomarqueurs inclut au moins CRP, sST2, TIMP-1 et TIMP-2 et dans lequel :
l'analyse des niveaux quantifiés inclut la détermination de si oui ou non le patient est sous risque en ce qui concerne le fait de présenter :
i) une décompensation de défaillance cardiaque ; et/ou
ii) un déclin rapide de symptômes cliniques de défaillance cardiaque ; et/ou
iii) une remodélisation ventriculaire ; et/ou
iv) des arythmies.

3. Procédé selon la revendication 2, l'échantillon biologique comprenant du plasma.
